# EUROPÄISCHE PATENTSCHRIFT

(11) **EP 1 735 431 B1**
(45) Veröffentlichungstag und Bekanntmachung des Hinweises auf die Patenterteilung: **16.09.2015**
(21) Anmeldenummer: 05729831.7
(22) Anmeldetag: 31.03.2005
(51) Int. Cl.: A61K 35/12

(54) **VERFAHREN ZUR EXPANSION POSTEMBRYONALER STAMM- UND PROGENITORZELLEN AUS NABELSCHNURBLUT UND IMMUNTHERAPEUTIKUM**
METHOD FOR EXPANDING POSTEMBRYONIC STEM AND PROGENITOR CELLS FROM UMBILICAL CORD BLOOD AND IMMUNOTHERAPEUTIC AGENT
PROCEDE D'EXPANSION DE CELLULES SOUCHES ET PROGENITRICES POST-EMBRYONNAIRES PROVENANT DU SANG DU CORDON OMBILICAL ET AGENTS THERAPEUTIQUES IMMUNITAIRES

(30) Priorität: 31.03.2004 WO PCT/EP2004/003429
(43) Veröffentlichungstag der Anmeldung: 27.12.2006
(73) Patentinhaber: IPD-Therapeutics B.V., 5228 DE's-Hertogenbosch (NL)
(72) Erfinder: Schwartz-Albiez, Reinhard, 69251 Galberg (DE); Punzel, Michael, 47652 Weeze (DE)
(74) Vertreter: Jansen, Cornelis Marinus
(86) Internationale Anmeldenummer: PCT/EP2005/003403
(87) Internationale Veröffentlichungsnummer: WO 2005/095584

(56) Entgegenhaltungen:
- DE-A- 10 245 927
- K THEUNISSEN ET AL.: "Long-term engrafting umbilical cord blood cells are preserved after ex vivo culture in strom-free culture" [Online] Mai 2001 (2001-05), KAREL A DICKE AND ARMAND KEATING , XP002332369 Gefunden im Internet: URL:http://mmserver.cjp.com/gems/blood/ABM T.10.Verfaillie.pdf> Seite 599 - Seite 603
- GUPTA PANKAJ ET AL: "Human LTC-IC can be maintained for at least 5 weeks in vitro when interleukin-3 and a single chemokine are combined with O-sulfated heparan sulfates: Requirement for optimal binding interactions of heparan sulfate with early-acting cytokines and matrix proteins" BLOOD, Bd. 95, Nr. 1, 1. Januar 2000 (2000-01-01), Seiten 147-155, XP002301975 ISSN: 0006-4971
- GUPTA PANKAJ ET AL: "Structurally specific heparan sulfates support primitive human hematopoiesis by formation of a multimolecular stem cell niche" BLOOD, Bd. 92, Nr. 12, 15. Dezember 1998 (1998-12-15), Seiten 4641-4651, XP002301976 ISSN: 0006-4971
- LEWIS IAN D ET AL: "Umbilical cord blood cells capable of engrafting in primary, secondary, and tertiary xenogeneic hosts are preserved after ex vivo culture in a noncontact system" BLOOD, Bd. 97, Nr. 11, 1. Juni 2001 (2001-06-01), Seiten 3441-3449, XP002301977 ISSN: 0006-4971
- M. SCHUBERT: "Einfluss regioselektiv modifizierter Heparansulfate auf den Erhalt und die Expansion primitiver hämatopoietischer Stammzelle und Vorläuferzellen" [Online] 2004, , HEIDELBERG , XP002332370 Gefunden im Internet: URL:http://www.doktor-schubert.de/download s/Dissertation%20M.Schubert.pdf> das ganze Dokument
- PUNZEL MICHAEL ET AL: "The microenvironment of AFT024 cells maintains primitive human hematopoiesis by counteracting contact mediated inhibition of proliferation." CELL COMMUNICATION & ADHESION. 2002 MAY-JUN, Bd. 9, Nr. 3, Mai 2002 (2002-05), Seiten 149-159, XP009038505 ISSN: 1541-9061
- GUPTA P ET AL: "Artificial "proteoglycan-like" molecules containing heparan sulfate enhance the ability of cytokines to maintain human hematopoietic stem cells in vitro" JOURNAL OF INVESTIGATIVE MEDICINE, Bd. 43, Nr. SUPPL. 2, 1995, Seite 342A, XP009038502 & CLINICAL RESEARCH MEETING; SAN DIEGO, CALIFORNIA, USA; MAY 5-8, 1995
- MOORE K A ET AL: "In vitro maintenance of highly purified, transplantable hematopoietic stem cells" BLOOD, W.B. SAUNDERS, PHILADELPHIA, VA, US, Bd. 89, Nr. 12, 1997, Seiten 4337-4347, XP002220989 ISSN: 0006-4971
- MOORE K A ET AL: "HEMATOPOIETIC ACTIVITY OF A STROMAL CELL TRANSMEMBRANE PROTEIN CONTAINING EPIDERMAL GROWTH FACTOR-LIKE REPEAT MOTIFS" PROCEEDINGS OF THE NATIONAL ACADEMY OF SCIENCES OF USA, NATIONAL ACADEMY OF SCIENCE. WASHINGTON, US, Bd. 94, April 1997 (1997-04), Seiten 4011-4016, XP002942328 ISSN: 0027-8424
- STRINGER SALLY E ET AL: "Identification of an MIP-1alpha-binding heparan sulfate oligosaccharide that supports long-term in vitro maintenance of human LTC-ICs." BLOOD, Bd. 101, Nr. 6, 15. März 2003 (2003-03-15), Seiten 2243-2245, XP002301974 ISSN: 0006-4971

## Beschreibung

### GEBIET DER ERFINDUNG

Die vorliegende Erfindung betrifft Verfahren zur ex vivo Expansion humaner post-embryonaler Stamm- und Progenitorzellen aus Nabelschnurblut. Insbesondere betrifft die Erfindung die Gewinnung, Vermehrung und Differenzierung von humanen postembryonalen Stamm- und Progenitorzellen zu immunkompetenten Zellen für deren Einsatz als Immuntherapeutika.

### HINTERGRUND DER ERFINDUNG

Verfahren zur Gewinnung und Züchtung von Stammzellen werden seit langem untersucht; siehe Punzel M et al, The microenvironment of AFT024 cells maintains primitive human hematopoiesis by counteracting contact mediated inhibition of proliferation, CELL COMMUNICATION & ADHESION (2002), 9, 149-159; Gupta P et al, Human LTC-IC can be maintained for at least 5 weeks in vitro when interleukin-3 and a single chemokone are combined with O-sulfated heparansulfates: Requirement for optimal binding interactions of heparin sulfate with early-acting cytokines and matrix proteins, BLOOD (2000) 95, 147-155; Gupta P et al, Artificial proteoglycan-like molecules containing heparin sulfate enhance the ability of cytokines to maintain human hematopoietic stem cells in vitro, JOURNAL OF INVESTIGATIVE MEDICINE (1995) 43, 342A, & CLINICAL RESEARCH MEETING; SAND DIEGO; CALIFORNIA; USA; MAY 5-8, 1995; Gupta P et al., Structurally specfic heparin sulfates support primitive human hematapoiesis by formation of a multimolecular stem cell niche, BLOOD (1998) 92, 4641-4651; Lewis ID et al, Umbilical cord blood cells capable of engrafting in primary secondary, and tertiary xenogeneic hosts are preserved after ex vivo culture in a noncontact system BLOOD (2001) 97, 3441-3449; Moore K et al., In vitro maintenance of highly purified transplantable hematopoietic stem cells, BLOOD (1991) 89, 4337-4347; Moore K et al., Hematopoietic activity of a stromal cell transmembrane protein containing epidermal growth factor like repeat motifs, PNAS USA (1997), 94, 4011-4016; Stringer SE et als Identifikation of an MIP-lalpha-binding heparin sulfate oligosaccharide that supports long-term in vitro maintenance of human LTC-ICs. BLOOD (2003) 101, 2243-2245);
Theunissen et al, Long-term engrafting umbilical cord blood cells are preserved after ex vivo culture in stroma-free culture (In: Autologous Blood and Marrow Transplantation: Chapter 14: pages 599-603 (2001): Carden Jennings Publishing Co. Ltd. (eds K.A. Dicke and A. Keating)); Schubert, M (*Einfluss regioselektiv modifizierter Herapansulfate auf den Erhalt und die Expansion primitiver hämatopoietischer Stammcellen und Vorläuferzellen:* Inauguraldissertation zur Erlangung des medizinischen Doktorgrades der medizinischen Fa cultät Heidelberg der Ruprecht-Karls-Universität, nach dem Prioritätsdatum veröffentlicht).
Theunissen et al., beschreiben die *Ex Vivo* Kultur von Nabelschnurblutzellen in Stroma-freien Kultur. Die Nabelschnurblutzellen sind kultiviert mit u.a. 6-O-sulfatierten Heparinen. Gupta Et Al., (1998; 2000), Kultivierung von-Knochenmark-Zellen in einem Stroma-freien System zusammen mit unter anderem schwefelhaltigen Heparinen. Schubert beschreibt den Einfluss regioselektiv modifizierter Herapansulfate auf den Erhalt und die Expansion primitiver hämatopoietischer Stammcellen und Vorläuferzellen.
Lewis ID et al. beschreiben in Blood (2001) 97, 3441-3449 *ex vivo* Kulturen mit und ohne Kontakt zu Stromazellen zum Langzeiterhalt und zur Züchtung (Expansion) angeblich pluripotenter Zellen aus Nabelschnurblut. Die Nabelschnurblut-Stammzellen werden in kollagenbeschichteten Kulturschalen gezüchtet, wobei die Stammzellen über eine Membran hinweg in fluidem Kontakt mit AFT024-Feederzellen stehen. Die AFT024-Feederzellen geben offenkundig einen unbekannten Faktor ab, der bei der Expansion der Stammzellen eine Arretierung der Differenzierung bewirkt. Ferner beschreiben sie Expansionskulturen in einem einheitlichen stromafreien MV8-Medium, das zusätzlich N-desulfatiertes O-sulfatiertes Heparin enthält. Die Kultur in dem stromafreien MV8-Medium mit dem N-desulfatierten O-sulfatierten Heparin erlaubt eine 180fache Expansion der TNC-Zellen, wobei aber die Vermehrung der CD34⁺-Zellen bzw. die CFC- und die LTC-IC-Expansion nur ein- bis zweifach ist. Bemerkenswert ist in diesem Zusammenhang, dass die Expansion der CD34⁺-Zellen im stromafreien MV8-Medium nach sieben Tagen nur das 1,1fache (± 0,2) ist und nach 14 Tagen nur das 2,0fache, bei einer Varianz von ± 2,0 (!!) (siehe Tabelle 2, Seite 3444), so dass nicht von einer Expansion der CD34⁺-Zellen ausgegangen werden darf. So vermehrte Stammzellen besitzen zudem nur mit Einschränkungen die Fähigkeit zur Selbsterneuerung und zur multilinearen Differenzierung zu myeloische und erythroide Zellen gemäß LTC-IC-Assay. So vermehrte Stammzellen können sich nicht zu lymphatische Zellen (NK- und NKT-Zellen) differenzieren.

Nachteilig am Stand der Technik ist somit, dass die Expansion der Stamm- und Progenitorzellen sehr gering ist und wegen der kleinen Zellzahlen so keine therapeutisch applizierbaren Therapeutika hergestellt werden können. Ferner werden die Stamm- und Progenitorzellen nicht so vermehrt, dass sie danach noch zu immunkompetente lymphatische Zellen differenzieren können.

### ZUSAMMENFASSUNG DER ERFINDUNG

Es ist Aufgabe der Erfindung, Verfahren und Mittel zur Expansion postembryonaler Stamm- und Progenitorzellen, vorzugsweise aus Nabelschurblut, bereitzustellen, das sich dadurch auszeichnet, dass nach ex *vivo*-Kultur und erfolgreicher Vermehrung sich die Zellen sowohl zu immunkompetente lymphatische NK- und NKT-Zellen als auch zu myeloische Progenitoren differenzieren können. Für den vorgesehenen Einsatz der Stamm- und Progenitorzellen als Therapeutikum ist erforderlich, dass die ex *vivo* Kultur und Expansion ohne das Beisein von Stroma- und Feederzellen erfolgen.

Diese Aufgabe wird dadurch gelöst, dass die postembryonalen Stamm- und Progenitorzellen in einem Medium expandiert werden, das neben üblichen Nährstoffen ein spezifisch regiomodifiziertes Glycan und/oder Glycosaminoglycan enthält, in dem insbesondere in ein oder mehreren Monomereinheiten das C₂-Atom *acyliert oder acetyliert ist* und das C₆-Atom O-sulfatiert ist. Ein Aspekt der Erfindung ist somit ein Verfahren zur Gewinnung und Expansion postembryonaler hämatopoletischer Stammzellen aus Nabelschurblut unter Vermeidung von unerwünschter Differenzierung, wobei Ausgangszellen aus Nabelschnurblut ex *vivo* in einem stromafreien Medium in Gegenwart eines regiomodifizierten Glycans und/oder Glycosaminoglycans gezüchtet werden, das so modifiziert ist, dass die Seitengruppe des C2-Atoms ein oder mehrerer Monomereinheiten des Glycans und/oder Glycosaminoglycans eine Acetyl- oder Acylgruppe mit 2 bis 12 Kohlenatomen aufweist; die Seitengruppe des C6-Atoms ein oder mehrerer Monomereinheiten des Glycans und/oder Glycosaminoglycans eine *6*-O-Sulfatgruppe ist und schließlich das Gewinnen von Stammzellen und Progenitorzellen, die zu myeloischen und lymphatischen Zellen differenzieren können. Das regiomodifizierte Glycan bzw. Glycosaminoglycan ist bevorzugt ausgewählt aus α1-4 Glycane, β1-3 Glycane, β1-4 Glycane, β1-3,β1-4 Glycosaminoglycane, β1-4, α1-4 Glycosaminoglycane, β1-4, β1-3, (α1-3) Glycosaminoglycane und β1-4, β1-3, (α1-4) Glycosaminoglycane. Das das reglomodifizierte Glycosaminoglycan kann ein Heparinderivat sein, das am C2-Atom im Wesentlichen N-desuffatiert und N-reacetyliert oder N-reacyliert wurde, das C6-O-Sulfatgruppen besitzt und das 5 Prozent oder weniger C3-O-Sulfat enthält. Besonders bevorzugt enthält das so regiomodifizierte Heparinderivat mindestens 60% C2-O-Sulfat und mindestens 80% C6-O-Sulfat. Das regiomodifizierte Glycan oder Glycosaminoglycan liegt erfindungsgemäß in dem Kulturmedium in einer Konzentration von 15 bis 50 mg/L vor.

Ein weiterer Aspekt der Erfindung liegt darin, dass die Stammzelleneigenschaften der so generierten Stammzellen und Progenitorzellen in einem ML-IC-Assay kontrolliert werden bzw in einem LY-IC-Assay und einem LTC-IC-Assay auf ihre lymphatischen oder myeloischen Eigenschaften. Die unter GMP-gerechten Bedingungen (GMP - *good manufacturing practice*) vermehrten Stamm- und Progenitorzellen können auch in funktionelle Lymphozyten (NK-Zellen und NKT-Zellen) differenziert werden.

Ein besonders wertvoller Aspekt der Erfindung betrifft die Herstellung einer therapeutischen Zusammensetzung, die erfindungsgemäße gewonnene und vermehrte Stamm- und Progenitorzellen enthält, bevorzugt zusammen mit einem pharmazeutisch akzeptablen Träger, Exzipienten oder anderem Verdünnungsmittel, beispielsweise für eine intraperitoneale, intervenöse, Intratekale oder andere Injektion.

Ein unmittelbarer Aspekt der Erfindung ist ferner ein Kulturmedium zur Expansion postembryonaler Stamm- und Progenitorzellen, das ein regiomodifiziertes Glycan und/oder Glycosaminoglycan enthält, wobei die Seitengruppe des C2-Atoms ein oder mehrerer Monomereinheiten des Glycans und/oder Glycosaminoglycans acyliert oder acetyliert ist, und die Seitengruppe des C6-Atoms ein oder mehrerer Monomereinheiten des Glycans und/oder Glycosaminoglycans eine 6-O-Sulfatgruppe ist, sowie die Verwendung derartig regiomodifizierten Glycane und Glycosaminoglycane zur Expansion postembryonaler Stamm- und Progenitorzellen.

Das hergestellte Therapeutikum kann direkt verabreicht werden zur Behandlung von Tumorerkrankungen, Vruserkrankungen, Hepatitis C, HIV, maligne Systemerkrankungen, akute Leukämien, chronische Leukämien, myeloproliferatives Syndrom (MPS), myelodysplastisches Syndrom (MDS), hochmaligne Non-Hodgkin-Lymphome (NHL), niedrigmaligne NHL, Morbus Hodgkin, multiples Myelom, Mb. Waldenström, Histiozytosis X, Amyloidose und solide Tumore wie Analkarzinom, Astrozytom, Basaliom, Bauchspeicheldrüsenkrebs, Blasenkrebs, Bronchialkarzinom, Brustkrebs, Corpuskarzinom, CUP-Syndrom, Darmkrebs, Dünndarmtumore, Eierstockkrebs, Endometriumkarzinom, Gallenblasenkrebs, Gebärmutterkrebs, Gebärmutterhalskrebs, Glioblastom, Himtumoren, Himlymphome, Himmetastasen, Hodenkrebs, Hypophysentumor, Karzinoide, Kehlkopfkrebs, Knochenkrebs, Kopf-Hals-Tumore, Kolonkarzinom, Kraniopharyngeome, Leberkrebs, Lebermetastasen, Lidtumor, Lungenkrebs, Magenkrebs, Medulloblastome, Melanom, Meningeome, Mycosis fungoides, Neurinom, Nierenkrebs, Non-Hodgkin-Lymphome, Oligodendrogliom, Ösophaguskarzinom, Ovarial-Karzinom, Pankreaskarzinom, Peniskrebs, Prostatakrebs, Rachenkrebs, Rektumkarzinom, Retinoblastom, Scheidenkrebs, Schilddrüsenkarzinom, Speiseröhrenkrebs, Spinaliom, Thymom, Urethrakrebs, Vulvakrebs, Weichteiltumoren, Zervixkarzinom.

Weitere bevorzugte Ausführungsformen und Aspekte der Erfindungen können den Ansprüchen, der Beschreibung und den Beispielen entnommen werden.

### BESCHREIBUNG DER ERFINDUNG

In der Beschreibung stehen "postembryonale Stammzellen" für somatische Stammzellen, insbesondere für Stammzellen aus humanem Nabelschurblut. Mit "Ausgangszellen" werden frisch aus dem Nabelschnurblut isolierte, nicht expandierte Stammzellen bezeichnet. Mit der Formulierung "unter Vermeidung von Differenzierung" wird verstanden, dass bei der *in vitro* Kultur die Stammzellen numerisch zumindest erhalten bleiben. Die Stammzelleigenschaft der vermehrten Zellen wird im ML-IC-Assay, wie nachstehend noch näher beschrieben, kontrolliert und bestätigt. Die durch Proliferation generierten, expandierten myeloischen und lymphatischen Progenitorzellen werden zudem im LTC-IC-Assay (myeloisch) und LY-IC-Assay (lymphatisch) quantifiziert, wie unten noch beschrieben.

Ein Aspekt der Erfindung ist somit ein Verfahren zur *in vitro* Generierung immunkompetenter Zellen (NK- und NKT-Zellen) durch Expansion postembryonaler Stamm- und Progenitorzellen aus Nabelschurblut. Das Verfahren zeichnet sich dadurch aus, dass die Ausgangszellen unter Vermeidung von unerwünschter Differenzierung in einem Medium gezüchtet werden, das ein regio- und stereoselektiv modifiziertes Glycan und/oder Glycosaminoglycan enthält, wobei die Modifikationen so sind, dass dieses Glycan N- und/oder O-gebundene Sulfat-, Amino-, Acetyl- und/oder Acylgruppen entlang der Polymerkette enthält. Besonders bevorzugt ist in ein oder mehreren Monomereinheiten das C₂-Atom acyliert oder acetyliert und das C₆-Atom O-sulfatiert. Mit anderen Worten, bei dem erfindungsgemäßen Verfahren werden die Nabelschurblut-Ausgangszellen unter Vermeidung von Differenzierung so vermehrt, dass einerseits die Stammzellen mindestens erhalten bleiben und parallel dazu primitive myeloisch- und lymphatisch-determinierte Progenitorzellen generiert werden. Ziel bei der Vermehrung ist also, dass bei einer Zellteilung zwei Stammzellen folgen, von der zumindest eine mit Stammzellfunktion erhalten bleibt und die andere sich zu einen definierten Progenitor differenziert oder, im Fall einer asymmetrischen Teilung, eine Stammzelle und eine Progenitorzelle entstehen, wobei sich die letztere zu immunkompetente NK- und NKT-Zellen differenzieren kann.

Dieser Effekt aus Stammzellerhalt beziehungsweise Stammzellvermehrung und Progenitorzellen-Generierung wird durch eine *in vitro* Kultivierung in einem Kulturmedium erreicht, das ein modifiziertes Glycan und/oder Glycosaminoglycan enthält, wobei in ein oder mehreren Monomereinheiten das C₂-Atom acyliert oder acetyliert und das C₆-Atom O-sulfatiert ist. Heparinmoleküle bestehen in der Regel aus unverzweigten Ketten von sulfatierten Saccharidbausteinen, die vor allem Glucosamin, Glucuronsäure und Iduronsäure enthalten; sie besitzen unterschiedliche Molekulargewichte zwischen 4 000 und 50 000 Dalton. Das durchschnittliche Gewicht der Heparine liegt bei 16 000 Dalton. Aufgrund der vielen im Molekül vorkommenden Carboxyl- und Sulfatreste ist Heparin stark negativ geladen und bildet daher unter physiologischen Bedingungen Komplexe mit basischen Proteinen. Ist das erfindungsgemäß modifizierte Glycan bzw. Glycosaminoglycan ein Heparinderivat, so müssen die Glucosamineinheiten zu 80% oder mehr eine 6-O-Sulfatgruppe besitzen. Eine selektive 6-O-Desulfatierung des Heparinderivats vermindert drastisch die Differenzierungsarretierung bei der Stammzellexpansion. Die 2-O-Sulfatgruppe der Iduronsäureeinheiten ist ferner wichtig für die Differenzierungsarretierung. Die 2-O-Sulfatgruppe sollte im Heparin zu 60% oder mehr vorhanden sein. Eine 2-O-De-sulfatierung vermindert signifikant die Arretierungsaktivität. Die N-Sulfatgruppen an den Glucosamineinheiten hingegen müssen entfernt und durch N-Acetyl oder N-Acylgruppen unterschiedlicher Länge, bevorzugt mit 3 bis 18 Kohlenstoffatomen, vorzugt 2 bis 12 und ganz besonders bevorzugt mit 3 bis 6 Kohlenstoffatomen ersetzt werden. Die N-Desulfatierung des Heparinderivats allein bewirkt keine Arretierungsaktivität. Im Heparinderivat sollten zudem die Glucosamine zu 5% oder weniger eine 3-O-Sulfatgruppe tragen. Das Molekulargewicht des modifizierten Heparinderivats liegt ideal bei ca. 10 kDa. Als Ausgangsquelle für die Produktion modifizierter Heparine kann Heparin aus intestinaler Schweine- oder aus Rindermukosa oder -niere verwendet werden. Erfindungsgemäße in Frage kommen ferner partialsynthetische Heparinoide aus Polysacchariden natürlichen Ursprungs. Der Fachmann kennt verschiedene weitere Quellen für Glycane, Glycosaminoglycane und sulfatierte heparinoide Polysaccharide, die nach entsprechender Modifikation vergleichbare oder äquivalente Strukturen aufweisen.

Nabelschnurblut ist eine vorteilhafte Quelle für somatische Stammzellen, denn gegen deren Verwendung bestehen kaum oder keine ethischen Bedenken. Zudem sind die humanen postembryonalen Stamm- und Progenitorzellen aus Nabelschurblut ontogenetisch naiv und sehr unreif, was für die angestrebten therapeutischen Ziele von großem Vorteil ist. Der klinisch therapeutische Einsatz dieser Zellen war wegen der geringen Zellzahlen in den meisten zur Verfügung stehenden Nabelschnur-Transplantaten hauptsächlich auf pädiatrische Patienten mit einem Körpergewicht von weniger als 40 kg beschränkt. Mit den bekannten stromafreien *in vitro* Verfahren konnten diese Stammzellen nicht expandiert werden und es war nicht möglich, mit ihnen ausreichend Immun-Effektorzellen zu generieren. Hauptproblem war die Induktion unkontrollierter Differenzierungen bei gleichzeitigem Verlust von Stammzellen aus der eingesetzten Ausgangspopulation.

Die bisherigen Erfahrungen zeigten, dass das nach der Abnabelung in der Plazenta verbliebene und über die Nabelschnur gewinnbare kindliche Restblut (CB oder *Cord Blood*) in der Regel genügend primitive blutbildende Stammzellen (HSC) enthält, um nunmehr auch bei Erwachsenen - und nicht nur bei Kindern - eine hämatologische und immunologische Rekonstitution zu erreichen. Die Verwendung von CB zur Stammzelltransplantation bei Erwachsenen war bislang wegen der nicht ausreichenden myeloischen und lymphatischen Progenitorzellzahl und insbesondere der Naivität der Immunzellen medizinisch limitiert, da zu lange hämatologische Rekonstitutionszeiten die Mortalität gegenüber der Knochenmarktransplantation signifikant erhöhen. Dabei bietet gerade CB den Vorteil, eine sie große Population von primitiven Stammzellen mit erhöhter Proliferations- und Stammzellpotenz enthalten. In letzter Zeit haben mehrere Berichte nachgewiesen, dass Stammzelltransplantationen aus Nabelschnurblut auch vorteilhaft bei allogenen Transplantationen von Patienten mit bösartigen Systemerkrankungen sind: Stammzellen aus Nabelschnurblut haben gegenüber solchen aus Knochenmark oder peripherem Blut entscheidende Vorteile: i) Sie sind schnell und unkompliziert ohne körperliche Belastung eines Spenders zu gewinnen. ii) Es besteht ein deutlich geringeres Risiko für die Übertragung viraler Erkrankungen (insbesondere des Cytomegalie-Viruses). iii) Das Risiko einer GvHD-Erkrankung (*graft versus host* disease) ist wesentlich geringer, so dass bei vergleichbaren Ergebnissen bis zu 3 HLA-Mismatches akzeptiert werden können. Nabelschnurblut-Präparate gibt es als sogenannte Fertigarzneimittel komplett vorgetestet und eingelagert und sie sind innerhalb kürzester Zeit verfügbar. Der therapeutische Einsatz für hämatologische Transplantationen war aber nicht nur wegen der geringen Zellzahl in den Transplantaten limititiert, sondern auch durch die lange Rekonstitutionsphase, welche entsprechenden klinischen Komplikationen nach der Transplantation zur Folge hatte. Diese Probleme werden durch das erfindungsgemäße Verfahren und die damit bereitgestellten Immuntherapeutika behoben.

Die Aktivität der immunologischen Effektorzellen aus Nabelschnurblut ist im Vergleich zu adulten Quellen zwar stark reduziert. Dies ist aber auch vorteilhaft, da es zu einer geringeren Inzidenz und Schwere einer GvHD beiträgt. Andererseits ergibt sich daraus eine erhöhte Morbidität und Mortalität durch Infektionskomplikationen bei Nabelschnurstammzelltransplantationen. Diese Probleme werden aber gleichfalls durch die erfindungsgemäße ex vivo Generierung von immunkompetenten Zellen gelöst.

Es wurde in mehreren Studien gezeigt, dass insbesondere die NK-Zellen bei allogenen Transplantationen eine wichtige Rolle spielen, in dem die alloreaktiven Spender-NK-Zellen nicht nur residuale Leukämiezellen sondern auch T-Zellen und Antigen-präsentierende Zellen des Wirtes abtöten und an der initialen Infektabwehr nach Transplantation teilhaben. Diese Immunreaktionen werden über Interaktionen sogenannter "Killer Immunoglobulin-like Rezeptoren" (KIR-Rezeptoren) und sogenannter Natürlicher Zytotoxizitäts-Rezeptoren (NCR) auf der Spender NK-Zelle mit bestimmten Antigenen des Haupt-Histokompatibilitätskomplexes der Klasse I (HLA KI. I) auf den Wirtszellen gesteuert.

Aus diesen Gründen ist eine ex *vivo* Expansion mit nachfolgender funktioneller Ausreifung primitiver CB-Progenitorzellen, insbesondere mit Differenzierungspotenzial zu NK-Zellen, für eine erfolgreiche Stammzelltransplantation wichtig. Dazu müssen wenige primitive Zellen expandiert werden, so dass ausreichend Progenitorzellen zur Verfügung stehen, um dann durch weitere Ausdifferenzierung - unter anderem zu NK- und NKT-Zellen - ein klinisch einsetzbares Therapeutikum zu generieren.

Bei der ex *vivo* Expansion humaner Stammzellen aus Knochenmark und CB wurden im Stand der Technik bislang unterschiedliche Kulturzusätze verwendet - unterschiedliche Kombinationen von Zytokinen, Stromazellfeederlayer, Bioreaktoren. Der Stand der Technik ist derzeit widersprüchlich oder vielfach nicht reproduzierbar. Es ist auch nicht klar, ob bei den beschriebenen *ex vivo* Kulturen zur Expansion der Zellzahl und zur Proliferationsinduktion die Stammzellen ihre Eigenschaften und Fähigkeit zur multilinealen Differenzierung und Selbsterneuerung beibehalten und zur Transplantation geeignet sind. Alle bisherigen *in vitro* Kultursysteme bewirken bei der Proliferation der Stammzellen gleichzeitig einen unkontrollierten Differenzierungsschub. Die alleinige Expansion der Zellzahl, phänotypischer Merkmale wie CD34 oder die Expansion von Progenitoren in liniendeterminierten Assays gibt keine Auskunft über die Stammzellen, ob diese nach noch zur regenerativen Selbsterneuerung und multilinealen Differenzierung fähig sind.

Die Schwierigkeit ist die Analyse der humanen Stammzellen im Allgemeinen und speziell der *in vitro* expandierten Stammzellen auf Zellzahl, Qualität und Differenzierungsgrad. Mehrere *in vitro* Assays sind etabliert, die primitive humane hämatopoetische Progenitorzellen analysieren: der Long-Term-Culture-lnitiating-Cell(LTC-IC)-Assay und der "cobblestone area forming cell assay" (CAFC). In Kombination mit dem "Limiting Dilution Assay" (LDA) erlauben sie eine Aussage über die Häufigkeit von LTC-IC oder CAFC. Das Problem ist jedoch, dass nach einer expandierten *in vitro* Kultur diese Assays keine Aussagen zur multilinealen (*multilineage*), das heißt, zur myeloischen, lymphatischen, erythrozytären und thrombozytären Selbsterneuerungs- und Differenzierungskapazität der noch vorhandenen primitiven Stammzellen erlauben - was aber für die Untersuchung der Effektivität der *in vitro* Kultur wichtig ist. Die Zahl und Charakteristik humaner Stammzellen wird bisher in sehr aufwendigen xenogenen Tiermodellen überprüft (*in utero-Schaf,* NOD-SCID-Maus). Eine Charakterisierung der expandierten Stammzellen auf der Basis einer Zelloberflächenmarkeranalyse ohne gleichzeitige funktionelle Testung ist nicht ausreichend verlässlich. Es hat sich im NOD-SCID-Maus-Modell gezeigt, dass ex *vivo* expandierte humane Stammzellen zwar die für primitive Stammzellen typischen Oberflächenmarker trugen, jedoch in der SCID-Maus nicht mehr zur Selbsterneuerung fähig waren.

Es gibt aber bislang keine Berichte für eine gerichtete Expansion und Generierung von Stammzellen aus Nabelschnurblut für den Einsatz als Therapeutikum. Es ist zwar bekannt, dass Komplexe von kationischem Chitosan und verschiedenen Glycosaminoglycanen, insbesondere Heparin und Chondroitinsulfat B als Festphase und unter Zusatz von Stammzellfaktor und IL-3 die Expansion von CD34⁺/CB-Zellen fördern können. Der Differenzierungsstand der CD34⁺-Zellen wurde aber nicht untersucht. Ferner war bekannt, dass Heparansulfate, synthetisiert von bestimmten Stromazellen, das Wachstum und die Differenzierung primitiver humaner hämatopoetischer Knochenmarksstammzellen (KM-HSC) beinflussen und dass solche KM-HSC ohne den Zusatz von Stromazellen nur in Gegenwart von bestimmten Glycosaminoglycanen *in vitro* für eine Zeitspanne von 5 Wochen bis maximal 80% erhalten werden können. Dabei wurde nur die 6-O-Sulfatierung des Heparins als das wesentliche strukturelle Element des Heparins beschrieben, welches diesen Effekt auslöst. Mit desulfatiertem-, N-sulfatiertem oder unmodifiziertem Heparin konnten die wachstumsfördernden Effekte nicht erzielt werden, was den spezifischen Effekt der erfindungsgemäßen Modifikationen unterstreicht. Es war zwar weiterhin bekannt, dass 6-O-sulfatierte Heparine ausschließlich myeloisch-determinierte Zellen (Langzeitkultur induzierende Zellen; LTC-IC) aus humanem Knochenmark bis zu 80% über 5 Wochen erhalten können. Eine Expansion des primitiven Stammzellpools erfolgte dabei aber nicht. Zudem wurde nicht untersucht, in wie weit derartige Heparine und Polysaccharide primitive und multipotente hämatopoietische Stammzellen in bezug auf Zellwachstum und Differenzierung in therapeutisch wirksame Immunzellen beeinflussen. Die Erfindung beruht auf der überraschenden Entdeckung, dass bestimmte regiospezifischen Modifizierungen an Glycan und/oder Glycosaminoglycanen wie an Heparin bei der ex vivo Expansionskultur postembryonaler Stamm- und Progenitorzellen aus CB die Differenzierung arretieren und somit eine nachfolgende Differenzierung in immunkompetente Zellen ermöglichen.

Das beanspruchte Verfahren erlaubt die Expansion primitiver postembryonaler Stammzellen aus einer kleinen Zellzahl bei zumindest numerischen Erhalt der Stammzellen und paralleler Generierung und Expansion von myeloischen und lymphatischen Progenitorzellen. Die Stammzellen können mit dem erfindungsgemäßen Verfahren *in vitro* so vermehrt werden, dass eine (Re)implantation der expandierten Zellen auch unter klinischen Bedingungen sowohl für die Regeneration der Hämatopoese als auch für den spezifischen Gewebs- und Organersatz angewendet werden kann. Das erfindungsgemäße Verfahren gewährleistet, dass reproduzierbar durch gezielten Einsatz von regio- und/oder stereoselektiv modifizierten Glycanen die Stammzellpopulation im Nabelschnurblut zur Proliferation angeregt wird, bei der die multipotenten Stammzellen erhalten bzw vermehrt werden und durch Zellteilungen myeloisch und lymphatisch determinierte Progenitoren expandiert beziehungsweise generiert werden.

Durch die erfindungsgemäße Anwendung der Glycane bzw. Glycosaminoglycane in einem sogenannten *"in vitro* Stammzellkultursystem" können therapeutisch einsetzbare Progenitorzellen aus postembryonalen Geweben expandiert bzw. generiert werden, die sowohl bei der Nabelschnurbluttransplantation eingesetzt werden können als auch als eigenständiges zelluläres Therapeutikum bei Tumorerkrankungen oder Viruserkrankungen (z.B. Hepatitis C oder HIV). Folgende Krankheitsentitäten kommen dabei in Betracht: maligne Systemerkrankungen, akute Leukämien, chronische Leukämien, myeloproliferatives Syndrom (MPS), myelodysplastisches Syndrom (MDS), hochmaligne Non-Hodgkin-Lymphome (NHL), niedrigmaligne NHL, Morbus Hodgkin, multiples Myelom, Mb. Waldenström, Histiozytosis X, Amyloidose und solide Tumore wie Analkarzinom,Astrozytom, Basaliom, Bauchspeicheldrüsenkrebs, Blasenkrebs, Bronchialkarzinom, Brustkrebs, Corpuskarzinom, CUP-Syndrom, Darmkrebs, Dünndarmtumore, Eierstockkrebs, Endometriumkarzinom, Gallenblasenkrebs, Gebärmutterkrebs, Gebärmutterhalskrebs, Glioblastom, Hirntumoren, Hirnlymphome, Hirnmetastasen, Hodenkrebs, Hypophysentumor, Karzinoide, Kehlkopfkrebs, Knochenkrebs, Kopf-Hals-Tumore, Kolonkarzinom, Kraniopharyngeome, Leberkrebs, Lebermetastasen, Lidtumor, Lungenkrebs, Magenkrebs, Medulloblastome, Melanom, Meningeome, Mycosis fungoides, Neurinom, Nierenkrebs, Non-Hodgkin-Lymphome, Oligodendrogliom, Ösophaguskarzinom, Ovarial-Karzinom, Pankreaskarzinom, Peniskrebs, Prostatakrebs, Rachenkrebs, Rektumkarzinom, Retinoblastom, Scheidenkrebs, Schilddrüsenkarzinom, Speiseröhrenkrebs, Spinaliom, Thymom, Urethrakrebs, Vulvakrebs, Weichteiltumoren, Zervixkarzinom.

Das erfindungsgemäße Verfahren beruht auf der Anwendung von definierten Glycanen beziehungsweise Glycosaminoglycanen durch regio- und/oder stereoselektive Modifikationen (Abbau, Ankopplung) funktioneller Seitengruppen (z.B. Carboxyl-, Sulfat-, Acetyl-, Acyl-, Aminogruppen) z.B. an bekannte Glycangerüste (α1-4 Glycane, β1-3 Glycane, β1-4 Glycane, β1-3, β1-4 Glycosaminoglycane, β1-3, β1-4, α1-3 Glycosaminoglycane und β1-4, α1-4 Glycosaminoglycane). Diese Verbindungen sind hinsichtlich (a) ihrer Molekülmasse, (b) ihres Sulfatierungsgrades und -musters, und (c) der Anzahl anderer vorstehend genannter funktioneller Seitengruppen und Verteilung der funktionellen Gruppen in der Monomereinheit und entlang der Polymerkette definiert.

Die Veränderungen der Seitengruppen zur erfindungsgemäßen Verwendung bestehen zum einen aus einer Modifikation einer Seitengruppe am C2-Atom der Monomereinheit des Glycans bzw. Glycosaminoglycans, vorzugsweise Desulfatierung und Reacylierung oder Reacetylierung, so dass definierte Verhältnisse der Seitengruppen am C2 Atom entlang der Polymerkette entstehen. Zum anderen ist die Anwesenheit einer Seitengruppe mit negativer Ladung, vorzugsweise eine 6-O-Sulfatgruppe, am C6 Atom der Monomereinheit des Glycans bzw. Glycosaminoglycans eine sterische Voraussetzung für das erfindungsgemäße Verfahren. Der Prozentsatz der insgesamt modifizierten Seitengruppen am C2 und/oder C6 Atom hängt von der jeweiligen Modifikation ab und der für das erfindungsgemäße Verfahren günstigste Prozentsatz kann mittels der im nachstehenden Beispiel genannten Assays vom Fachmann leicht ermittelt werden.

Diese Verbindungen können als Zusatz in Expansions- und Differenzierungskulturen postembryonaler Stammzellen für sämtliche klinische und industrielle Anwendungen verwendet werden. Einer der wesentlichen Vorteile der Erfindung liegt im Einsatz einzelner chemisch genau definierter Substanzen zur reproduzierbaren Expansion und Generierung postembryonaler Progenitorzellen

In den zu der vorliegenden Erfindung führenden Experimenten wurden primitive hämatopoetische Stammzellen *in vitro* unter Zusatz von erfindungsgemäßen Polysacchariden und definierten Cytokinen ohne die Kokultivierung mit Stromazellen oder anderen Feederzellen in ihrem ursprünglichen primitiven Zustand erhalten und gleichzeitig gerichtete Progenitorzellen generiert. Das Differenzierungspotential dieser Zellen wurde in anschließenden Kulturen auf Einzelzellebene nachgewiesen. Die eingesetzten Polysaccharide mit definierten funktionellen Gruppen zeichnen sich durch ihren definierten Anteil an 6-O-Sulfat- und N-Sulfatgruppen, 2-O- und 3-O-Sulfatgruppen, sowie N-acetyl bzw N-acyl Gruppen aus. Es handelt sich dabei um regio- und stereoselektiv sulfatierte und acetylierte/acylierte Wiederholungseinheiten von Polysacchariden mit unterschiedlich einstellbaren definierten Durchschnittssubstitutionsgraden für die einzelnen Gruppen. Durch die Konstellation der jeweiligen Seitengruppen mit unterschiedlichem Charakter (hydrophil, z.B. -SO₃; hydrophob, z.B. -acyl) und definierter Verteilung entlang der Polymerkette werden die hydrophoben und hydrophilen Verhältnisse innerhalb des Glycans beziehungsweise Glycosaminoglycans wesentlich verändert.

Somit betrifft die vorliegende Erfindung ein Verfahren zur selektiven Vermehrung postembryonaler Stammzellen unter Vermeidung von Differenzierung, dadurch gekennzeichnet, dass die Ausgangszellen in einem Medium gezüchtet werden, dass ein modifiziertes Glycan und/oder Glycosaminoglycan enthält, wobei die Modifikation so ist, dass die ursprüngliche Seitengruppe des C2 Atoms einer oder mehrerer Monomereinheiten des Glycans und/oder Glycosaminoglycans modifiziert ist und die Seitengruppe des C6 Atoms einer oder mehrerer Monomereinheiten des Glycans und/oder Glycosaminoglycans eine Seitengruppe mit negativer Ladung, vorzugsweise eine 6-O-Sulfatgruppe ist.

Vorzugsweise ist das Glycan und/oder Glycosaminoglycan ein α1-4 Glycan, β1-3 Glycan, β1-4 Glycan, β1-3, β1-4-Glycosaminoglycan, β 1-3, β1-4, α1-3 Glycosaminoglycan und/oder β1-4, α1-4 Glycosaminoglycan. Bevorzugt ist das C2-Atom ein oder mehrerer Monomereinheiten desulfatiert und eine neue Seitengruppe ist am C2-Atom eingeführt, vorzugsweise eine Acetyl-, Acyl- (vorzugsweise Butyl-), Amino- oder CarboxymethylGruppe. Besonders bevorzugt ist eine Acetyl- oder Acyl-Gruppe. Unter Reacetylierung wird die Ankopplung einer Acetylgruppe an das C2 Atom der Monomereinheit verstanden.

Der vorteilhafteste Prozentsatz der jeweils modifizierten C2 und/oder C6 Atome kann mittels der nachstehend beschriebenen funktionellen Assays bestimmt werden.

Bei den für die vorliegende Anmeldung verwendeten Kohlenhydraten-Grundstrukturen handelt es sich um Verbindungen wie sie von der "IUPAC-IUB Joint Commission on Biochemical Nomenclature (JCBN) definiert wurden (Eur. J. Biochemistry 126 (1982), 439-441) und beispielsweise von S.Dumitriu (Polysaccharides in Medical Applications, Marcel Dekker, 1996) sowie J.Lehmann (Kohlenhydrate, Chemie und Biologie, G.Thieme Verlag, 1996) beschrieben wurden. Dabei werden unter dem Ausdruck Glycane alle Oligo- und Polysaccharide zusammengefasst, die von ihrem sequentiellen Aufbau sowohl Homoglycane, bestehend aus einer Monosaccharidwiederholungseinheit, als auch Heteroglycane, bestehend aus verschiedenen Monosaccharidwiederholungseinheiten, sein können.

Als Oligosaccharide/Polysaccharide werden Verbindungen zusammengefasst, in denen Monosaccharide durch glycosidische Bindungen miteinander verknüpft sind. Oligosaccharide und Polysaccharide mit repetitiven Saccharidsequenzen werden hier als Glycane zusammengefasst. Der Begriff "Oligosaccharid" bezeichnet Glycane, die aus 2 bis 10 Monosaccharideinheiten bestehen, während der Begriff "Polysaccharid" alle Glycane umfasst, die aus mehr als 10 Monosaccharideinheiten bestehen.

Als Glycosaminoglycane werden Heteroglycane mit repetitiven Disaccharideinheiten jeweils bestehend aus einem Aminozucker und einer Uronsäure definiert, die sich untereinander hinsichtlich ihrer Monosaccharideinheiten und ihrer glycosidischen Bindung unterscheiden.

Die für das erfindungsgemäße Verfahren verwendbaren Glycane bzw. Glycosaminoglycane können unterschiedliche Verknüpfungs- und Verzweigungsgrade aufweisen, d.h. z.B. linear, verzweigt oder zyklisch (z.B. ß-Cyclodextrin) sein. Hinsichtlich ihres Molekulargewichts unterliegen die für das erfindungsgemäße Verfahren verwendbaren Glycane bzw. Glycosaminoglycane keiner Einschränkung, allerdings ist offensichtlich die biologische Aktivität bei größeren Molekülen besser. Günstige Molekulargewichte liegen im Bereich von etwa 10 bis 35 kD oder darüber.

Der Fachmann kann mittels allgemein bekannter Verfahren, z.B. über klassische Schutzgruppentechniken und/oder Festphasensynthese die für das erfindungsgemäße Verfahren geeigneten Poly-/Oligosaccharide herstellen. Verfahren zum Entfernen bzw. Ankoppeln von Seitenketten, z.B. zur regio- und stereoselektiven Modifikation sind ebenfalls allgemein bekannt; siehe z.B. Baumann et al., Carbohydrate Res. 308 (1998), 391-388; 331 (2001), 43-57; Baumann et al., Carbohydrate Res. 337 (2002), 1297-1307, Baumann et al., Macromol. Chem. Phys. 201 (2000), 1950-1962; Deutsches Patent 4444445.1; Huppertz et al., In: Frontiers in Biomedical Polymer Applications (Eds. Ottenbrite, Chiellini, Cohn, MigliaFresi, Sunamoto), Band 2, Seiten 115-129, Technomic 1999; Grootenhuis et al., Nat. Struct. Biol. 2 (1995), 736-739; Westerduin et al., Bioorg. Med. Chem. 2 (1994), 1267-1280; Wessel et al., Carbohydr. Res. 204 (1990), 131-139; Matsuo et al., Carbohydr. Res. 241 (1993), 209-215; Kurita et al., Macromolecules 31 (1998), 4764-4769; Kariya et al., J. Biol. Chem. 275 (2000), 25949-25958; Du et al., Carbohydr. Res. 329 (2000), 17-24; und Groth und Wagenknecht, Biomaterials 22 (2001), 2719-2729.

Der Fachmann kennt auch geeignete Züchtungsverfahren und Medien, um post-embryonale Stammzellen kultivieren zu können; siehe z.B. DE 196 08 813 C2 oder EP-B1 0 695 351 bzw. das nachfolgende Beispiel. Der Fachmann kann auch anhand einfacher Versuche die optimale Konzentration des modifizierten Glycans bzw. Glycosaminoglycans für den gewünschten Zweck bestimmen. Das "Grundmedium", das u.a. ein erfindungsgemäßes modifiziertes Glycan bzw. Glycosaminoglycan enthält kann jedes Medium sein, das üblicherweise für die Stammzellexpansion verwendet wird. In einer bevorzugten Ausführungsform des erfindungsgemäßen Mediums ist das "Grundmedium", in dem u.a. ein erfindungsgemäßes Glycan bzw. Glycosaminoglycan enthalten ist, IMDM ("Iscoves Modifiziertes Dulbeccos Medium"; Invitrogen). Die gewünschten Stammzellen werden in dem vorstehenden Medium unter geeigneten Bedingungen, gegebenenfalls unter (teilweiser) Erneuerung des Mediums in geeigneten Zeitabständen, gezüchtet. Geeignete Bedingungen, beispielsweise hinsichtlich geeigneten Behältern, Temperatur, relativer Luftfeuchte, O₂-Gehatt und CO₂-Gehalt der Gasphase sind dem Fachmann bekannt. Vorzugsweise werden die Zellen in dem vorstehenden Medium unter den folgenden Bedingungen kultiviert: (a) 37°C, (b) 100% rel. Luftfeuchte, (c) 10% O₂ und (d) 5% bis 7% CO₂. Der Fachmann kann auch die gewünschte Steuerung der Differenzierung der tierischen Zellen anhand üblicher Kriterien (morphologische Kriterien, Anwesenheit bzw. Abwesenheit spezifischer Oberflächenproteine etc.; siehe dazu auch die Assays im nachstehenden Beispiel) überwachen.

Eine weitere bevorzugte Ausführungsform des erfindungsgemäßen Verfahrens betrifft die Isolierung und/oder Anreicherung und/oder selektive Vermehrung von postembryonalen Stammzellen unter Verwendung eines 2-O-desulfatierten Heparins, N-desulfatierten und/oder N-desulfatierten und reacetylierten Heparin enthaltenden Mediums. Dabei erfolgt die Züchtung bzw. Haltung der Zellen wie vorstehend bzw. in dem nachstehenden Beispiel beschrieben.

Der Fachmann kann anhand einfacher Versuche die für das erfindungsgemäße Expansionsverfahren geeigneten Konzentrationen der modifizierten Glycane bzw. Glycosaminoglycane ermitteln. In einer bevorzugten Ausführungsform des erfindungsgemäßen Expansionsverfahrens ist das modifizierte Glycan bzw. Glycosaminoglycan in einer Konzentration von 2 bis 50 mg/l im Kulturmedium vorhanden, wobei eine Konzentration von 10 mg/ml am meisten bevorzugt ist.

Für das erfindungsgemäße Züchtungsverfahren können folgende nicht-embryonale, humane Stammzellen verwendet werden: somatische Stamm- oder Progenitorzellen, wobei hämatopoetische Stammzellen bevorzugt sind. Der Fachmann kennt geeignete Quellen zur Gewinnung und zur Expansion dieser Stammzellen. Hämatopoetische Stammzellen können z.B. aus fötaler Leber, Nabelschnurblut oder Knochenmark erhalten werden, wobei die Gewinnung aus Nabelschnurblut für das erfindungsgemäße Verfahren bevorzugt ist.

In einer weiteren bevorzugten Ausführungsform des erfindungsgemäßen Verfahrens erfolgt im Anschluß an die Züchtung ein funktioneller Nachweis der Stamm- und Progenitorzelleigenschaften. Die nachstehende Beispiele erläutert die Erfindung.

### Beispiel 1- Expansionskultur hämatopoetischer Progenitorzellen mittels modifizierter Heparine als Zusatz zum Züchtungsmedium.

Stammzellangereicherte Nabelschnurblutzellen wurden in einem definierten und klinisch applizierbaren Expansionssystem unter Zusatz erfindungsgemäßer Glycane mit Bestimmung der Expansion primitiver myeloischer als auch lymphatischer Progenitorzellen als Zeichen der Stammzellvermehrung (quantifizierbar als ML-IC; "Myeloid-Lymphoid-Initiating Cells") für 5 Wochen kultiviert.

Das Kulturmedium bestand aus "Iscoves modifiziertem Dulbeccos Medium" (Invitrogen, Karlsruhe) mit 12,5% fötalem Kälberserum und 12,5% Pferdeserum (Stem Cell Technologies, Vancouver, Canada). Dazu wurden die in Tabelle 1 beschriebenen, sterilisierten Glycane mit funktionellen Seitengruppen in einer Konzentration von 20 mg/l sowie folgende Zusätze supplementiert: 2 mmol/l L-Glutamin (Invitrogen), Penicillin (1000 U/ml), Streptomycin 100 U/ml (Invitrogen), 10⁻⁶ mmol/l Hydrocortison, 25 µM 2-Mercaptoethanol-B, 10 pg/ml GM-CSF (Immunex Corp., Seattle, WA), 250 pg/ml G-CSF (Amgen, Thousand Oaks, CA), 200 pg/ml SCF (Stem Cell Technologies), 50 pg/ml LIF (Stem Cell Technologies), 200 pg/ml MIP-1 alpha (Stem Cell Technologies), und 50 pg/ml IL-6 (Stem Cell Technologies), 10 ng/ml Flt-3L und 10 ng/ml Thrombopoietin (Immunex Corp., Seattle, WA).

Die Kulturen wurden bei 37°C und 5% CO₂ mit 48stündigem Mediumwechsel für 5 Wochen erhalten, anschließend wurde die Expansion der stammzelläquivalenten multipotenten Zellen im ML-IC Assay (Nachweis von LTC-IC und NK-IC) ermittelt.

ML-IC-Assay: Zur Testung der Stammzelleigenschaften der ex *vivo* kultivierten Nabelschnurblutzellen wurde der ML-IC-Assay (Punzel et al., Blood 93 (1999), 3750-3756) verwendet. Die Zellen der Primärkulturen wurden durch mechanische Pipettierung gleichmäßig in Suspension gebracht und anschließend wurde der Inhalt jeder individuellen Primärkultur (200 µl) in 4 neue, bereits präetablierte AFT024-stromaabhängige Sekundärkulturen gebracht (50 µl Primärsuspension pro Sekundärkultur), und zwar in der Art und Weise, dass jede der 4 Sekundärkulturen einer primären Einzelzelle sich in der gleichen Position in den neuen Microtiterplatten befand. Damit wurde gewährleistet, dass alle sekundären Progenitoren sich der primären Einzelzelle zuordnen lassen. Zwei der Sekundärkulturen wurden dabei unter myeloischen Bedingungen im sekundären LTC-IC-Assay über 5 Wochen kultiviert. Die anderen zwei Kulturen wurden unter lymphatischen *in vitro* Differenzierungsbedingungen ebenfalls für weitere 5-7 Wochen im LY-IC-Assay kultiviert und anschließend auf reife funktionelle NK-Zellen wie unten beschrieben untersucht.

LTC-IC-Assay (Gupta et al., Blood 95 (2000), 147-155): Frisch sortierte Einzelzellen (Tag 0) oder die gesamte Nachkommenschaft einer Einzelzelle (Tag 14) wurden in AFT024 Kulturen mit Langzeitkultur (LTBMC)-Medium (IMDM/12,5% FCS/12.5% Pferdeserum sowie Penicillin/Streptomycin und 10⁻⁶ mmol Hydrocortison) kultiviert (1 x Mediumwechsel alle 7 Tage). Nach 5 Wochen wurde das Medium entfernt und die Kultur mit semisolidem Methylzellulose-Medium (1,12% Methylzellulose, IMDM/30% FCS, 3IU/ml Erythropoetin, 7,5% zytokinhaltigem Überstand der Zellinie 5637 ATCC HB-5) versehen. Einzelzellen mit der Fähigkeit sekundäre kolonieformende Zellen (CFC) zu generieren wurden per Definition als LTC-IC bezeichnet.

LY-IC Assay: Frisch sortierte Einzelzellen (Tag 0) oder die gesamte Nachkommenschaft einer Einzelzelle (Tag 14) wurden in AFT024 Kulturen in lymphatischem Differenzierungsmedium (DMEM/Ham's F 12-Medium 2:1 (V/V) mit 20% humanem hitzeinaktiviertem AB-Serum sowie 20 mg/ml Ascorbinsäure, 50 µmol Selen, 25 µmol Mercaptoäthanol, 50 µmol Äthanolamin, 1000 U/ml IL-2, 5 ng/ml IL-3 [nur am Tag 0], 10 ng/ml Flt-3L, 10 ng/ml SCF und 20 ng/ml IL-7) kultiviert. Nach 5-7 Wochen wurden alle Kulturen mit visuell objektivierbarer klonaler Zellproliferation durch mechanische Pipettierung geerntet und mittels FACS-Analyse auf reife NK-Zellen (CD56+/CD3-), NKT-Zellen (CD3+/CD56+) und auch Pro-B-Zellen (CD 19+/CD56-) untersucht.

Primär kultivierte Einzelzellen, die in der Lage waren sowohl LTC-IC als auch lymphatische Effektorzellen zu generieren, sind dann stammzelläquivalente ML-IC. Die Expansion der Nabelschnurblutzellen errechnet sich aus dem Verhältnis von unreifen LTC-IC und NK-IC vor und nach der Expansionskultur. Die Ergebnisse der vorstehenden Untersuchungen sind in Tabelle 1 dargestellt.

**TABELLE 1**

| Expansion | H0 | H0-1 | H1 | H2 | H3 | H3-1 | C1 | C2 |
|---|---|---|---|---|---|---|---|---|
| Myeloisch | 1,0 | 0,9 | 3,9 | 10,7 | 11,8 | 4,1 | 0,3 | 2,0 |
| Lymphatisch | 1,0 | 1,1 | 15,2 | 11,7 | 12,4 | 5,0 | 7,4 | 7,7 |

| | | | | | | | | |
|---|---|---|---|---|---|---|---|---|
| HO: Standard unmodifiziertes Heparin mit geringem 3-*O*-Sulfatgehalt, H0-1 Standard unmodifiziertes Heparin mit erhöhtem 3-O-Sulfatgehalt (Ausgangsheparine für regioselektive Modifizierungen); H1: 2-O-desulfatiertes Standard Heparin, aus H0; H2: N-desulfatiertes Standard Heparin aus Heparin H0; H3: N-desulfatiertes und reacetyliertes Standard Heparin aus H0; H3-1: N-desulfatiertes und reacetyliertes Standard Heparin aus H0-1; C1: Chitosan N-Sulfatiert; C2: Chitosan N-desulfatiert und N-acetyliert. | | | | | | | | |

Bei den Heparinen ist 2-O-Sulfat die Sulfatgruppe am C2 der Iduronsäureeinheit, 3-O-Sulfat die Sulfatgruppe am C3 der Glucosamineinheit, 6-O-Sulfat die Sulfatgruppe am C6 der Glucosamineinheit, 2-N-Sulfat und N-Acetat die N-Sulfat und N-Acetylgruppe jeweils am C2 der Glucosamineinheit. Das durchschnittliche Molekulargewicht der eingesetzten Heparine lag bei 10-12 kDa.

Als Chitosan-Ausgangsmaterial wurde Chitosan aus Krabben von FLUKA mit einem Molekulargewicht von ca. 150 kDa genommen und durch Hydrolyse in Fragmente von 29 kDa gespalten. Mit diesem hydrolyiserten Ausgangs-Chitosan wurde keine Expansion der Stammzellkulturen erreicht (Ergebnisse nicht gezeigt).

Die eigentliche Stammzellexpansion ergibt sich aus der kombinierten Erhöhung der myeloischen und lymphatischen Expansionsfähigkeit der in den oben beschriebenen Expansionsbedingungen manipulierten CB-HSC. Der Zusatz von regio- und stereoselektiv modifizierten Glycanen zeigt im Vergleich zu den Kulturen mit unmodifiziertem Heparin (H0 und H0-1) einen signifikanten Expansionseffekt. Diese Ergebnisse machen deutlich, dass für den durch modifizierte Glycane erzeugten Effekt auf die Expansion von CB- HSC eine Desulfatierung am C2-Atom mit Reacetylierung bzw. Acylierung verantwortlich ist.

**TABELLE 2**

| Regioseletivmodifiziertes Glycan | 2-*O*-Sulfat | 3-*O*-sulfat | 6-*O*-Sulfat | 2-*N*-Sulfat | 2-*N*-Acetyl | 2-N-CH₂-C00H |
|---|---|---|---|---|---|---|
| H0 | 60% | 5% | 90% | 90% | 7% | - |
| H0-1 | 58% | 33% | 90% | 88% | 5% | - |
| H1 | 9% | 6% | 85% | 90% | 5% | - |
| H2 | 60% | 0% | 85% | 0% | 14% | - |
| H3 | 60% | 5% | 85% | 0% | 100% | - |
| H3-1 | 58% | 33% | 83% | 0% | 100% | - |
| C1 | - | 0% | 70% | 88% | 12% | 38% |
| C2 | - | 0% | 0% | 59% | 41%* | 0% |

Tabelle II ist zu entnehmen, dass bei Heparinen erfindungsgemäß im Gesamtpolymer zu >80% eine 6-O-Sulfatgruppe an der Glucosaminmonomereinheit notwendig ist. Eine selektive O-6-Desulfatierung gemäß H.Baumann et al., Carbohydrate Res (1998) 308, 381-388 resultiert in einer drastischen Reduzierung der funktionellen Aktivität bei der Stammzellexpansion. Die 2-O-Sulfatgruppe der Iduronsäuremonomereinheit trägt ebenfalls zur Aktivität bei und sollte zu >60% des Gesamtpolymers vorhanden sein. O-2-Desulfatierung reduziert ebenfalls signifikant die Aktivität. Die N-Sulfatgruppe der Glucosaminmonomereinheit muss gänzlich eliminiert und ersetzt werden durch eine N-acetyl oder N-Acylgruppe unterschiedlicher Länge. Die N-Desulfatierung allein gibt keine erhöhte Aktivität. Die 3-O-Sulfatgruppe der Glucosaminmonomereinheit sollte bei weniger als oder gleich 5% des Gesamtpolymers liegen. Das Molekulargewicht der modifizierten Heparine sollte ideal bei ca. 10 kDa liegen. Als Ausgangsquelle für die Produktion modifizierter Heparine kann Heparin aus intestinaler Schweine- oder aus Rindermukosa oder -niere verwendet werden. Als Ausgangsmaterial für die Versuche diente Natriumheparinat aus Schweinemukosa, reinst für Forschungszwecke geeignet, Aktivität 178,000 IU/g (SERVA, Heidelberg, Germany). Die übrigen Chemikalien waren, sofern nicht anders angegeben, reine Substanzen der Firmen Aldrich, Fluka oder Sigma.

Ein Beispiel für ein funktionell aktives modifiziertes Heparin ist ein N-desulfatiertes, N-reacetyliertes Heparin mit folgender Struktur.

### Modifiziertes Heparin mit veränderter Sulfatierung und Acetylierung

| Heparin | 2-O-S | 3-O-S | 6-O-S | C2-N-S | N-Ac | O-Ac | NH2 |
|---|---|---|---|---|---|---|---|
| | 63 % | 5% | 84% | 0% | 100% | 0% | 0% |

Die Charakterisierung der Heparinderivate erfolgte mittels 13C-NMR-Spektroskopie bei 75 MHz. Die Signale wurden identifiziert gemäß B.Casu et al., Arzneimittelforschung 33, 135-142, 1983; E.A.Yates et al., Carbohydrate Res. 294, 15-27, 1996. Die Quantifizierung der verschiedenen Sulfatgruppen erfolgte gemäß Casu et al., Arzneimittelforschung 33, 135-142, 1983. Die Molekulargewichte wurden säulenchromatographisch bestimmt. Die Säulen wurden kalibriert mit Keratansulfaten definierter Molekulargewichte [H. Butz et al. J. Biol. Chem. 267, 34023408, 1992]. Regioselektive Modifizierung am C3 und C6 der Glucosamin-Monomereinheit, N-Acetylierung und N-Carboxymethylierung am C2 der Glucosamin-Monomereinheit erfolgten gemäß Baumann H und Faust V, Regioselektive Modifikationen der Chitosane, Carbohydrate Res. (2001) 331, 43-57.

### Beispiel 2 - Herstellung eines Immuntherapeutikums

Zur GMP-gerechten (good manufacturing practice) Anwendung wurden vorexpandierte Zellen in Suspensionskultur weiter expandiert unter folgenden Bedingungen:
DMEM/Ham's 12-Medium 2:1 (V/V) mit 10% humanem hitzeinaktiviertem AB-Serum sowie 20 mg/ml Ascorbinsäure, 50 µmol Selen, 25 µmol Mercaptoethanol, 50 µmol Ethanolamin, 1000 U/ml IL-2, 10 ng/ml Flt-3L, 10 ng/ml SCF, 20 ng/ml IL-7, 10ng/ml IL-15 und 10 ng/ml IL-21) kultiviert. Nach 3 bis 4 Wochen wurden die Zellen durch mechanische Pipettierung geerntet und mittels FACS-Analyse auf reife funktionelle NK-Zellen wie folgt untersucht:
   a) durch durchflusszytometrische Analyse der vorhandenen Oberflächenantigene. Dabei wurden über Mehrfarbenanalyse die CD56 (N-CAM) und CD16 (FcRγIII) positiven, CD3-negativen Zellen als NK-Zellen definiert und diese auf ihre KIR- und Aktivator Rezeptoren analysiert. Diese Zellen waren sowohl für spezifische KIR-Rezeptoren CD158 (CD158a (KIR2DL1),b (KIR2DL3)) beziehungsweise CD94/CD159a (NKLG2a) als auch für bestimmte Nkp-Aktivator-Rezeptoren positiv, so dass eine zelllytische NK-Zellaktivität über Bindung an die entsprechenden HLA Klasse I-Antigene auf der nativen Zielzelle als molekulare Voraussetzung der Zelllyse gewährleistet war.
   b) Zusätzlich wurde die lytische Aktivität der generierten NK-Zellen direkt über einen Standard ⁵¹Cr-Release Assay auf K562 Zellen als Zielzelle nachgewiesen. Die durch den Zusatz unterschiedlicher Polysaccharide generierten multipotenten Stammzellen zeigten im weiteren Differenzierungskulturverlauf keine qualitativen Unterschiede hinsichtlich der Expression der oben erwähnten Oberflächenantigene, der Unterschied war ausschließlich in der Zellzahl der expandierten Zellen zu sehen.

Alternativ können die expandierten Progenitoren auch in vivo zur Ausreifung gebracht werden durch zusätzliche subkutane Administration von IL-2 im Patienten (Miller et al., 2005)

Damit steht ein zelluläres Therapeutikum zur Verfügung, welches Patienten als Blutprodukt über eine Vene transfundiert werden kann. In dem zellulären Produkt sind die benannten expandierten Natürlichen Killerzellen bzw deren Progenitoren enthalten, die dann therapeutische Effekte beim Patienten hervorrufen. Diese sogenannten Natürlichen-Killer-Zell-Therapeutika (NKZT) können sowohl im Zusammenhang mit einer Stammzell- bzw. Knochenmarktransplantation appliziert werden als auch als eigenständige Therapeutika zur Behandlung von bösartigen Erkrankungen des blutbildenden und lymphatischen Systemes, und sämtlicher anderer bösartiger Erkrankungen einsetzbar. Ein weiterer Vorteil dieser Zellen ist die Passage der Bluthirnschranke, was auch den Einsatz bei allen bösartigen Hirntumoren ermöglicht.

Weiterhin ist auch ein Einsatz zur Behandlung von chronischer Viruserkrankungen (z.B. Hepatitis C und HIV) möglich.

### Beispiel 3 - Produktion und Charakterisierung modifizierter Heparine

Gewinnung von Heparinen mit niedrigem 3-O-Sulfatgehalt mittels AT III-Affinitätschromatographie.

Anti-Thrombin III (AT III) bindet an eine spezifische Heparinsequenz, dessen Grundstruktur als ein Pentasaccharid mit einer 3-O-Sulfogruppe an einem inneren Glucosamin definiert ist, wobei die Sulfogruppe für die Bindung von ATIII unbedingt erforderlich ist (R.Linhard and I.Capila, Angew. Chemie 2002, 114, 426-450). Da erfindungsgemäß modifzierte Heparine einen sehr geringen Anteil an 3-O-Sulfogruppen an der Glucosaminmonosaccharideinheit aufweisen müssen, wurde eine ATIII-Affinitätschromatographie auf einer Sepharose CL4B Säule angewendet, um im Durchlauf AT III nichtbindende Heparine für deren weitere chemische Modifikation zu erhalten. Das hierzu verwendete AT III wurde aus humanem Blutplasma isoliert.

Die Trennung von AT III-bindenden von den AT III-nichtbindenden Heparinen wurde über biomagnetische Separation kontrolliert. Dazu wurden magnetische AT III-Beads hergestellt, um in einer magnetischen Säule die AT III-bindende Fraktion der Heparine von solchen zu trennen, die kein AT III binden. AT III wurde mittels Biotin-X-NHS (Calbiochem) biotinyliert. Magnetische, mit Strepavidin benetzte Beads (Dynabeads M-270 Streptavidin; Dynal) wurden in einem Verhältnis von 1 ml Beads auf 200 µg des ATIII Biotins eingesetzt.. Nach 30-minütiger Inkubation auf dem Rüttler wurden die Magnetobeads erneut in der Magnetsäule mit PBS gespült, um das ungebundene ATIII-Biotin zu entfernen. Die so gewonnenen ATIII-Magnetobeads konnten nun für den ATIII-Bindungs-Assay verwendet werden.

### AT III-Bindungsassay.

Jeweils 1ml der ATIII-Magnetobeads wurde mit 1 ml des zu untersuchenden Heparansulfates bei Zimmertemperatur 30 Minuten auf dem Rüttler inkubiert. Das Gemisch wurde in einen Magnetständer gegeben, der Überstand mit dem ungebundenen Anteil Heparansulfat abgehoben und die Heparansulfat-Konzentration photometrisch bei 232 nm ermittelt; hierzu wurden vorab für jedes Heparin Eichkurven erstellt. Der verbliebene, an die ATIII-Magnetobeads gebundene Anteil wurde nun 10 Minuten mit 2M NaCl auf dem Rüttler inkubiert und so die ATIII-Bindung gelöst. Dann wurde erneut im Magnetständer der Über-stand mit dem Heparansulfat abgenommen, das im ersten Schritt an das ATIII gebunden hatte. Dessen Konzentration wurde dann photometrisch bei 232 nm mittels der Eichkurve bestimmt.

Die so gewonnen 3-O-Sulfat-armen Heparine wurden dann nach etablierten Methoden partiell oder ganz modifiziert; siehe Nagasawa K et al., Glucosamin N-desulfation, Carbohydrate Res. (1977) 58, 47-55; Danishefsky, I et al., Glucosamin N-reacetylation, Arch Biochem Biophys (1960) 90, 114-121; M.Höök et al., Glucosamin N-desulfation, Anal Biochem. (1982) 119, 236-245, und wie vorstehend in Beispiel 2 beschrieben.

## Patentansprüche

1. Verfahren zur Gewinnung und Expansion postembryonaler hämatopoietischer Stammzellen aus Nabelschurblut unter Vermeidung von erwünschter Differenzierung, wobei aus Nabelschnurblut isolierte Stammzellen, ex vivo in einem stromafreien Medium in Gegenwart eines regiomodifizierten Glycans und/oder Glycosaminoglycans gezüchtet werden, das wie folgt modifiziert ist: die Seitengruppe des C2-Atoms ein oder mehrerer Monomereinheiten des Glycans und/oder Glycosaminoglycans weist eine Acetyl- oder Acylgruppe auf; die Seitengruppe des C6-Atoms ein oder mehrerer Monomereinheiten des Glycans und/oder Glycosaminoglycans ist eine 6-O-Sulfatgruppe, und Gewinnen von generierten Stammzellen und Progenitorzellen, die gezielt zu myeloischen und lymphatischen Zellen differenzieren können.

2. Verfahren nach Anspruch 1 , wobei das regiomodifizierte Glycan bzw. Glycosaminoglycan ausgewählt ist aus: α1-4 Glycane, β1-3 Glycane, β1-4 Glycane, β1-3,β1-4 Glycosaminoglycan, β1-4, α1-4 Glycosaminoglycan und β1-4, β1-3, (α1-3) Glycosaminoglycan ist.

3. Verfahren nach einem der Ansprüche 1 oder 2, wobei das regiomodifizierte Glycan oder Glycosaminoglycan in einer Konzentration von 15 bis 50 mg/L im Medium vorhanden ist.

4. Verfahren nach einem der Ansprüche 1 bis 3, wobei die Eigenschaften der Stammzellen in einem ML-IC-Assay kontrolliert werden.

5. Verfahren nach einem der Ansprüche 1 bis 4, wobei die vermehrten Stamm- und Progenitorzellen in funktionelle Lymphozyten (NK-Zellen und NKT-Zellen) differenziert werden.

6. Kulturmedium zur Expansion postembryonaler Stamm- und Progenitorzellen, **dadurch gekennzeichnet**, das es ein regiomodifiziertes Glycan und/oder Glycosaminoglycan enthält, wobei die Seitengruppe des C2-Atoms ein oder mehrerer Monomereinheiten des Glycans und/oder Glycosaminoglycans acyliert oder acetyliert ist, und die Seitengruppe des C6-Atoms ein oder mehrerer Monomereinheiten des Glycans und/oder Glycosaminoglycans eine 6-O- Sulfatgruppe ist.

7. Verwendung von regiomodifizierten Glycanen und Glycosaminoglycanen, wobei die Seitengruppe des C2-Atoms ein oder mehrerer Monomereinheiten des Glycans und/oder Glycosaminoglycans acyliert oder acetyliert ist, und die Seitengruppe des C6 Atoms ein oder mehrerer Monomereinheiten des Glycans und/oder Glycosaminoglycans eine 6-0-Sulfatgruppe besitzt, zur Expansion postembryonaler Stamm- und Progenitorzellen.

## Claims

1. Method for obtaining and expanding postembryonic, haematopoietic stem cells from umbilical cord blood, while avoiding undesired differentiation, wherein stem cells isolated from umbilical cord blood are cultured ex vivo in a stroma-free medium in the presence of a regio-modified glycan and/or glycosaminoglycan which is modified as follows: the side group of the C2 atom of one or more monomer units of the glycan and/or glycosaminoglycan has an acetyl or acyl group; the side group of the C6 atom of one or more monomer units of the glycan and/or glycosaminoglycan is a 6-0-sulphate group, and obtaining generated stem cells and progenitor cells which can differentiate in a targeted manner into myeloid and lymphatic cells.

2. Method according to claim 1, wherein the regio-modified glycan or glycosaminoglycan is selected from: α1-4 glycans, β1-3 glycans, β1-4 glycans, β1-3, β1-4 glycosaminoglycan, β1-4, α1-4 glycosaminoglycan and β1-4, β1-3, (α1-3) glycosaminoglycan.

3. Method according to one of claims 1 or 2, wherein the regio-modified glycan or glycosaminoglycan is present in a concentration of 15 to 50 mg/l in the medium.

4. Method according to one of claims 1 to 3, wherein the properties of the stem cells are checked in an ML-IC assay.

5. Method according to one of claims 1 to 4, wherein the multiplied stem and progenitor cells are differentiated into functional lymphocytes (NK cells and NKT cells).

6. Culture medium for expanding postembryonic stem and progenitor cells, **characterised in that** it contains a regio-modified glycan and/or glycosaminoglycan, wherein the side group of the C2 atom of one or more monomer units of the glycan and/or glycosaminoglycan is acylated or acetylated, and the side group of the C6 atom of one or more monomer units of the glycan and/or glycosaminoglycan is a 6-0-sulphate group.

7. Use of regio-modified glycans and glycosaminoglycans, wherein the side group of the C2 atom of one or more monomer units of the glycan and/or glycosaminoglycan is acylated or acetylated, and the side group of the C6 atom of one or more monomer units of the glycan and/or glycosaminoglycan has a 6-0-sulphate group, for expanding postembryonic stem and progenitor cells.

## Revendications

1. Procédé pour l'obtention et l'expansion de cellules souches hématopoïétiques post-embryonnaires, provenant du sang du cordon ombilical, tout en évitant une différenciation souhaitable, dans lequel des cellules souches isolées du sang du cordon ombilical sont cultivées ex vivo dans un milieu sans stroma, en présence d'un glycanne et/ou d'un glycosaminoglycanne régio-modifié, qui est modifié comme suit : le groupe latéral de l'atome C2 d'une ou de plusieurs unités monomères du glycanne et/ou du glycosaminoglycanne présente un groupe acétyle ou acyle ; le groupe latéral de l'atome C6 d'une ou plusieurs unités monomères du glycanne et/ou du glycosaminoglycanne est un groupe 6-O-sulfate, et en obtenant des cellules souches et de cellules progénitrices générées, qui peuvent d'une manière ciblée se différencier en cellules myéloïdes et lymphatiques.

2. Procédé selon la revendication 1, dans lequel le glycanne ou le glycosaminoglycanne régio-modifié est choisi parmi les α1-4-glycannes, les β1-3-glycannes, les β1-4-glycannes, le β1-3,β1-4-glycosaminoglycanne, le β1-4- α1-4-glycosaminoglycanne et le β1-4,β1-3,(α1-3)-glycosaminoglycanne.

3. Procédé selon l'une des revendications 1 ou 2, dans lequel le glycanne ou le glycosaminoglycanne régio-modifié est présent dans le milieu à une concentration de 15 à 50 mg/L

4. Procédé selon l'une des revendications 1 à 3, dans lequel les propriétés des cellules souches sont contrôlées dans un essai ML-IC.

5. Procédé selon l'une des revendications 1 à 4, dans lequel les cellules souches et progénitrices multipliées sont différenciées en lymphocytes fonctionnels (cellules NK et cellules NKT).

6. Milieu de culture pour l'expansion de cellules souches et progénitrices post-embryonnaires, **caractérisé en ce qu'**il contient un glycanne et/ou un glycosaminoglycanne régio-modifié, le groupe latéral de l'atome C2 d'une ou de plusieurs unités monomères du glycanne et/ou du glycosaminoglycanne étant acylé ou acétylé, et le groupe latéral de l'atome C6 d'une ou plusieurs unités monomères du glycanne et/ou du glycosaminoglycanne étant un groupe 6-O-sulfate.

7. Utilisation de glycannes et de glycosaminoglycannes régio-modifiés, pour laquelle le groupe latéral de l'atome C2 d'une ou plusieurs unités monomères du glycanne et/ou du glycosaminoglycanne est acylé ou acétylé, et le groupe latéral de l'atome C6 d'une ou plusieurs unités monomères du glycanne et/ou du glucosaminoglycanne possède un groupe 6-O-sulfate, pour l'expansion de cellules souches et progénitrices post-embryonnaires.
